# EUROPÄISCHE PATENTANMELDUNG

(11) **EP 3 842 855 A1**
(43) Veröffentlichungstag der Anmeldung: **30.06.2021**
(21) Anmeldenummer: 21157838.0
(22) Anmeldetag: 08.05.2010
(51) Int. Cl.: G02C 13/00, A61B 5/11, A61B 5/00, G02C 7/02

(54) **VERFAHREN, VORRICHTUNG UND COMPUTERPROGRAMM ZUR ZENTRIERDATENERMITTLUNG**

(30) Priorität: 17.06.2009 DE 102009025215
(62) Teilanmeldung aus: 18188534.4
(71) Anmelder: Carl Zeiss Vision International GmbH, 73430 Aalen (DE)
(72) Erfinder: Kubitza, Matthias, 73434 Aalen (DE); Gamperling, Michael, 89340 Leipheim (DE); Cabeza-Guillén, Jesús-Miguel, 73434 Aalen (DE)
(74) Vertreter: Glawe, Delfs, Moll

(57) **Zusammenfassung**

Gegenstand der Erfindung ist ein Verfahren zur Zentrierdatenermittlung, bei dem
a. eine tatsächliche momentane Kopfhaltung eines Probanden bestimmt wird,
b. eine Anordnung einer Brillenfassung (202) relativ zur bestimmten tatsächlichen momentanen Kopfhaltung ermittelt wird,
c. mit Hilfe eines automatisierten Verfahrens eine habituelle Kopfhaltung bestimmt wird, und
d. die bestimmte tatsächliche momentane Kopfhaltung mit Hilfe der bestimmten habituellen Kopfhaltung korrigiert wird, wobei die bestimmte tatsächliche momentane Kopfhaltung mit Hilfe der bestimmten habituellen Kopfhaltung derart korrigiert wird, dass die Zentrierdaten auf Basis der habituellen und nicht der zufälligen Kopfhaltung bestimmt werden.

Erfindungsgemäß ist vorgesehen, dass
e. die Bestimmung der habituellen Kopfhaltung durch Ermittlung einer bevorzugten Kopfhaltung aus Daten einer Aufzeichnung der Kopfhaltung über eine Zeitspanne, während der der Proband abgelenkt ist, erfolgt, und dass
f. die bestimmte tatsächliche momentane Kopfhaltung mit Hilfe der bestimmten habituellen Kopfhaltung korrigiert wird, indem die Abweichung der momentanen Kopfhaltung zur habituellen Kopfhaltung ermittelt wird und anhand dieser eine Umrechnung der Zentrierwerte erfolgt.

Die Erfindung betrifft ferner eine Vorrichtung und ein Computerprogrammprodukt zur Durchführung des Verfahrens.

## Beschreibung

Die Erfindung betrifft ein Verfahren zur Ermittlung der habituellen Kopfhaltung eines Probanden nach dem Oberbegriff des Patentanspruchs 1 sowie eine Vorrichtung zur Ermittlung der habituellen Kopfhaltung eines Probanden nach dem Oberbegriff des Patentanspruchs 13. Schließlich betrifft die Erfindung ein Computerprogramm zur Durchführung der vorstehend angegebenen Verfahren.

Wenn Augenoptiker zur optischen Anpassung einer Brille die Zentrierdaten, also alle notwendigen Angaben zur korrekten Zentrierung von Brillengläsern in einer Brillenfassung für den individuellen Anwendungszweck des Kunden oder Probanden, ermittelt, so kann wie folgt verfahren werden:
Der Kunde wird gebeten eine möglichst natürliche Kopf- und Körperhaltung einzunehmen. Die Festlegung der Zentrierwerte erfolgt dann durch Markierung der Durchblickpunkte in der Scheibenebene der Brillenfassung mittels Stift oder mittels Videoaufnahme und anschließender manueller (wie z.B. bei der von der Anmelderin unter der Marke "Video Infral" vertriebenen Videozentriereinrichtung) bzw. automatisierter (wie z.B. bei der von der Anmelderin unter der Marke "RV Terminal" vertriebenen Videozentriereinrichtung) Zentrierdatenermittlung.

Zur Ermittlung des Vorneigungswinkels ist ein Hilfsmittel Namens "Y-Stick" bekannt, welches in einem Zeitpunkt den Vorneigungswinkel der Brillenfassung "einfriert", damit dieser anschließend abgelesen werden kann. Der so erfasste Vorneigungswinkel wird in einem nachfolgenden Schritt dazu benutzt, den Kunden in gleicher Position vor einem Zentriersystem zu positionieren.

Weiterhin ist aus der EP 1 591 064 A1 ein Messgerät zur Bestimmung der Kopfneigung bekannt, welches von der Anmelderin der EP 1 591 064 unter der Bezeichnung "Ysis" vertrieben wird. Dieses Messgerät wird an der Brillenfassung befestigt. Dieser Vorschlag zielt darauf ab, die "normale" Kopfhaltung zu einem Zeitpunkt jeweils für Nah- und Fernblickrichtung festzuhalten. Aus den Kopfneigungen, die beim Blick in die Ferne sowie beim Lesen eingenommen werden, wird der Unterschied der Kopfneigung zwischen Fern- und Nahsehaufgaben ermittelt. Daraus werden dann die zugehörigen Durchblickpunkte durch das Brillenglas bestimmt.

Sämtliche vorstehenden Methoden sind statisch, d.h. zur Ermittlung einer habituellen Kopfhaltung wird eine "Momentaufnahme" der jeweiligen Situation herangezogen.

Die WO 01/62139 A1 offenbart, das Verhalten eines Brillenträgers hinsichtlich seiner Blickgewohnheiten zu klassifizieren. Es wird die typische Kopf- und Augenbewegung bei verschiedenen Sehaufgaben bestimmt. Davon abhängig, wird das Blickverhalten des Probanden kategorisiert nach "Beweglichkeit" der Augen und des Kopfes ("head/eye mover"). Die Ergebnisse der Analyse werden dabei als Grundlage für die Auswahl eines geeigneten Brillenglases verwendet.

Aus der EP 1 747 750 A1 ist es bekannt, die häufigsten Blickrichtungen relativ zum Kopf festzustellen. Zu diesem Zweck werden Kopfhaltung und Blickrichtung gleichzeitig detektiert. Aus den ermittelten relativen Blickrichtungen wird die relative "Netto"-Blickrichtung und der daraus resultierende Durchblickpunkt durch ein Brillenglas bestimmt.

Die DE 2004 063 160 A1 beschreibt ein Verfahren und eine Einrichtung zum Anpassen einer Brille, insbesondere zur Erfassung der Habitus-Kopfhaltung eines Probanden, dessen bzw. deren Messdaten Eingang finden bei der Video-Zentrierdaten-Bestimmung. Bei dem Verfahren werden vom Probanden mehrere Video-Fotos zur Ermittlung der Kopfhaltungswinkel eines Probanden in verschiedenen Kopfhaltungspositionen gefertigt, die Kopfhaltungswinkel erfasst und mit dem Vorneigungswinkel der Brillenfassung sowie dem Hornhautscheitelabstand abgeglichen. Die entsprechenden Video-Fotos werden in einem Moment aufgenommen, wenn nach Meinung des Bedienenden der Proband seine natürliche Habitus-Kopfhaltung eingenommen hat.

Eine Zentriermessung, also eine Messung, bei der alle notwendigen Angaben zur korrekten Zentrierung von Brillengläsern in einer Brillenfassung für den individuellen Anwendungszweck des Kunden oder Probanden, ermittelt werden, stellt für den Kunden des Optikers eine erzwungene Situation dar. Zwar ist aus der Zentriermessung bekannt, wie der Kunde während einer Aufnahme den Kopf hielt, diese Kopfhaltung entspricht allerdings nicht zwangsläufig seiner "normalen", d.h. häufigsten Kopfhaltung in tatsächlich ungezwungenen Situationen.

Dies ändert sich auch nicht, wenn das in der DE 10 2004 063 160 A1 beschriebene Verfahren zum Einsatz kommt. Darüber hinaus birgt dieses Verfahren die Gefahr, dass gar nicht die vermeintlich natürliche Kopfhaltung aufgenommen wird, sonder eine andere, weil der Proband innerhalb der Reaktionszeit der Bedienenden bereits eine andere Kopfhaltung eingenommen hat.

Die Aufgabe der Erfindung besteht nunmehr darin, ein verbessertes Verfahren zur Ermittlung der habituellen Kopfhaltung eines Probanden bereitzustellen, und zwar dergestalt, dass die ermittelte Kopfhaltung der tatsächlichen "normalen" Kopfhaltung des Probanden näher kommt, als dies bei den oben beschriebenen Verfahren zum Stand der Technik der Fall ist. Eine weitere Aufgabe der Erfindung besteht darin, eine entsprechende Vorrichtung zur präziseren Ermittlung der habituellen Kopfhaltung eines Probanden bereitzustellen. Die erstgenannte Aufgabe wird durch ein gattungsgemäßes Verfahren zur Ermittlung der habituellen Kopfhaltung eines Probanden mit den Merkmalen des kennzeichnenden Teils des Patentanspruchs 1 gelöst.

Die danach angegebene Aufgabe wird durch eine gattungsgemäße Vorrichtung zur Ermittlung der habituellen Kopfhaltung eines Probanden mit den Merkmalen des kennzeichnenden Teils des Patentanspruchs 13 gelöst.

Vorteilhafte Ausführungen und Weiterbildungen der Erfindung sind Gegenstand der Unteransprüche.

Das erfindungsgemäße Verfahren zur Ermittlung der habituellen Kopfhaltung eines Probanden umfasst die folgenden Verfahrensschritte:
a. Aufzeichnung der Kopfhaltung über eine gewisse Zeitspanne und
b. Ermittlung einer bevorzugten Kopfhaltung aus der aufgezeichneten Kopfhaltung.

Die entsprechende Vorrichtung zur Ermittlung der habituellen Kopfhaltung des Probanden umfasst in korrespondierender Weise:
a. eine Aufzeichnungseinrichtung zur Aufzeichnung der Kopfhaltung über die Zeitspanne und
b. eine Ermittlungseinrichtung zur Ermittlung der bevorzugten Kopfhaltung aus der aufgezeichneten Kopfhaltung.

Durch das vorstehend skizzierte Verfahren bzw. die insbesondere zur Durchführung dieses Verfahrens ausgebildete entsprechende Vorrichtung wird vermieden, dass die habituelle Kopfhaltung des Probanden irrtümlich als eine zufällig zu einem Zeitpunkt aufgenommene bzw. bestimmte Kopfhaltung angenommen wird, die gerade nicht seiner gewöhnlichen Kopfhaltung entspricht, wie dies nach den in der Beschreibungseinleitung beschriebenen Verfahren aus dem Stand der Technik der Fall sein kann. Die eingangs gestellte Aufgabe wird durch dieses Verfahren bzw. die entsprechende Vorrichtung also vollumfänglich gelöst.

Die Annahme der habituellen Kopfhaltung als die aus einer über eine gewisse Zeit hinweg aufgezeichneten Kopfhaltung ermittelte bevorzugte Kopfhaltung ermöglicht es z.B. auch, die bei der Zentriermessung (z.B. mit Hilfe der unter den Bezeichnungen Relaxed Vision Terminal und Video Infral von der Anmelderin vertriebenen Videozentriergeräten) gemessene Kopfhaltung zu korrigieren, so dass die Zentrierdaten auf Basis der "normalen" und nicht der zufälligen Kopfhaltung bestimmt werden.

Die Erfindung umfasst auch ein Verfahren zur Zentrierdatenermittlung, bei dem
a. eine tatsächliche momentane Kopfhaltung eines Probanden bestimmt wird,
b. eine Anordnung einer Brillenfassung relativ zur bestimmten tatsächlichen momentanen Kopfhaltung ermittelt wird,
c. eine mit einem automatisierten Verfahren habituelle Kopfhaltung bestimmt wird und
d. die bestimmte tatsächliche momentane Kopfhaltung mit Hilfe der automatisiert bestimmten habituellen Kopfhaltung korrigiert wird.

In entsprechender Weise ist Gegenstand der Erfindung auch eine Vorrichtung zur Zentrierdatenermittlung mit
a. einer Kopfhaltungsbestimmungseinrichtung, um die tatsächliche momentane Kopfhaltung des Probanden zu bestimmen,
b. einer Ermittlungseinrichtung, um die Anordnung der Brillenfassung relativ zur bestimmten tatsächlichen momentanen Kopfhaltung zu ermitteln,
c. einer Vorrichtung zur automatisierten Bestimmung der habituellen Kopfhaltung und
d. einer Korrektureinrichtung zur Korrektur der bestimmten tatsächlichen momentanen Kopfhaltung mit Hilfe der bestimmten habituellen Kopfhaltung.

Bei dem erfindungsgemäßen Verfahren zur Ermittlung der habituellen Kopfhaltung eines Probanden kann die Zeitspanne, innerhalb der die Kopfhaltung aufgezeichnet wird, vorgegeben werden. So kann zum Beispiel die Kopfhaltung nur während der Zeit aufgezeichnet werden, während der sich auch verlässliche Daten zur habituellen Haltung des Kopfes des Probanden entnehmen lassen. Die Aufzeichnung erzwungener und/oder unnatürlicher Kopfhaltungen kann vermieden werden. Darüber hinaus lässt sich die Zeitspanne individuell so festlegen, dass das aufgenommene Datenmaterial eine hinreichend verlässliche Aussage über die tatsächliche habituelle Kopfhaltung zulässt. Günstig ist es z.B., wenn aus der Aufzeichnung nur solche Zeitabschnitte zur Ermittlung der habituellen Kopfhaltung ausgewählt werden, innerhalb derer der Proband auch eine Kopf- und/oder Körperhaltung eingenommen hat, die ergonomischen Grundsätzen entspricht.

Als bevorzugte Kopfhaltung ist die Kopfhaltung zu verstehen, in der der Proband seinen Kopf bei einer vorgegebenen Sehaufgabe mit einer gewissen Wahrscheinlichkeit hält. Typische Werte für diese Wahrscheinlichkeit sind z.B. über 30 %, über 50 %, über 80 % oder gar über 90 % für die bestimmte Sehaufgabe. Eine derartige Analyse lässt sich mit üblichen mathematischen/statistischen Methoden durchführen. Geeignete Software Algorithmen können bei Bedarf automatisch oder mit Unterstützung durch den Benutzer Extremwerte (Ausreißer) aus der aufgezeichneten Datenmenge der habituellen Kopfhaltung eliminieren, bevor eine Bestimmung einer typischen Kopfhaltung erfolgt. Es erfolgt dann z.B. ein Auswahl aus der Menge dieser wahrscheinlichsten Kopfhaltungen.

Als bevorzugte Kopfhaltung kann auch die aufgezeichnete Haltung des Kopfes gewählt werden, in der der Proband den Kopf mit der größten Wahrscheinlichkeit hält.

Obwohl es grundsätzlich möglich ist, dem Probanden freizustellen, in welcher Körperhaltung und/oder was er bei der Aufzeichnung seiner Kopfhaltung betrachtet, ist es vielfach günstig, diesem bestimmte Sehaufgaben aufzuerlegen. Insbesondere wenn die Kopfhaltung Eingang in die Brillenglasberechnung oder die Zentrierung eines Brillenglases findet, ist es sinnvoll, besondere Aufmerksamkeit den Blickrichtungen für die Nähe und/oder die Ferne zu widmen. Die Erfindung sieht z.B. vor, als bevorzugte Kopfhaltung eine der aufgezeichneten Haltungen zu wählen, in der der Proband den Kopf beim Blick in die Nähe und/oder beim Blick in die Ferne hält.

Weiterhin ist es sinnvoll, die Körperhaltung des Probanden zu beachten. Beim Blick in die Ferne wird eine Person eher stehen, wohingegen eine Person beim Blick in die Nähe, wie z.B. beim Lesen, bevorzugt eine sitzende Position einnimmt. Die Erfindung sieht daher vor, als bevorzugte Kopfhaltung eine der aufgezeichneten Haltungen zu wählen, während der der Proband steht und/oder sitzt.

Die habituelle Kopfhaltung hängt in besonderem Maß von der Tätigkeit des Probanden und den visuellen Eindrücken, die der Proband wahrnimmt ab. Es ist daher weiter günstig, dem Probanden konkrete Alltagssituationen und/oder alltägliche Sehaufgaben darzubieten unter denen seine habituelle Kopfhaltung bestimmt werden soll. Der hiermit verbundene Vorteil liegt darin, dass der Proband/Kunde von der eigentlichen Messsituation abgelenkt ist und damit keine unnatürliche Kopfhaltung einnimmt. Die Sehaufgaben können darin bestehen, dass der Proband/Kunde sich z.B. im Laden des Optikers, der die habituelle Kopfhaltung aufzeichnet frei bewegt und z.B. die Auslagen betrachtet oder dass er z.B. Verkehrssituationen verfolgt, die über ein elektronisches Medium, wie ein Display, einen Beamer usw., dargestellt werden, wie dies z.B. in der US 2007/0229761A1 beschrieben ist oder dass er gebeten wird ein Schriftstück durchzulesen. Die Sehaufgaben werden z.B. so gewählt, dass eine typische Kopfhaltung im Stehen und/oder im Sitzen bestimmt werden kann. Bei der Zentrierdatenermittlung für eine Lesebrille wird z.B. die habituelle Kopfhaltung zu berücksichtigen sein, während der der Proband sitzt und liest.

Konkret können dem Probanden/Kunden zur dynamischen Ermittlung der habituellen Kopfhaltung über ein Zeitintervall Alltagssituationen bzw. alltägliche Sehaufgaben im Nah- und/oder Fernbereich dargeboten werden. In dieser Zeit werden die Kopfhaltung, ggf. in Kombination mit der zugehörigen Körperhaltung und ggf. auch die (relative) Position wie z.B. die Neigung der Brille bzw. der Brillengläser, die der Proband trägt, fortlaufend gemessen. Die Messung erfolgt so, dass der Proband/Kunde davon nicht beeinträchtigt wird. Im Anschluss an die Messung kann die jeweils häufigste Kopfhaltung, soweit mit aufgezeichnet auch die zugehörige Körperhaltung- und ggf. auch die Position, insbesondere Neigung der Brille bzw. der Brillengläser bestimmt und später bei der Ermittlung der Zentrierdaten berücksichtigt werden. Insbesondere können die Durchblickpunkte bei Nah- und/oder Fernsehaufgaben, der sogenannte Inset und/oder die Korridorlänge bestimmt werden. Konkret kann z.B. aus der Kopfhaltung beim Sehen im Nah- und Fernbereich bei bekannter Position des Sehziels der Durchblickpunkt durch das Brillenglas bestimmt werden. Sind die Durchblickpunkte bekannt, kann damit auch die Größe des Insets bestimmt werden, sowie die Länge des Korridors des Nahteils. Ein eventueller Unterschied zwischen Kopfrichtung und Blickrichtung kann bei der Bestimmung der Durchblickpunkte und/oder des Insets mit berücksichtigt werden.

Die Kopfhaltung des Probanden kann auf unterschiedlichste Art und Weise ermittelt werden. So kann z.B. die Aufzeichnung der Kopfhaltung mittels eines am Kopf des Probanden angebrachten Positionsaufzeichnungsgeräts durchgeführt werden. Dieses Positionsaufzeichnungsgerät kann mittelbar also z.B. am Brillenfassungsbügel oder unmittelbar am Kopf des Klienten befestigt sein. Dieses Positionsaufzeichnungsgerät kann nach dem Prinzip eines Datenloggers mit anschließendem Datentransfer über eine Basisstation funktionieren oder der Datentransfer kann zeitgleich über eine drahtlose Verbindung, wie WPAN (Wireless Personal Area Network), nämlich z.B. via Bluetooth, oder WLAN (Wireless Local Area Network) online während des gesamten Messintervalls erfolgen. Das Positionsaufzeichnungsgerät kann entweder absolute Lageinformationen messen oder aber Änderungen in der Lage. Im zweiten Fall muss der Messwertaufnehmer vor und/oder nach dem Messvorgang in eine Referenzlage gebracht werden, deren Ausrichtung bekannt ist. Als Referenzlage kann z.B. eine horizontale Tischfläche oder die Auflage einer speziell dafür vorgesehenen Halterung dienen.

Die Aufzeichnung der Kopfhaltung kann zusätzlich oder alternativ mittels eines Bildaufzeichnungsgeräts durchgeführt werden. Ein derartiges Bildaufzeichnungsgerät kann z.B. ein Video- oder Digitalkamerasystem mit angeschlossener automatisierter Bildauswertung sein. Die Bilddaten können entweder als zweidimensionale (2D) Daten direkt oder nach Erstellung eines dreidimensionalen (3D) Modells als 3D-Daten weiterverarbeitet werden. Aus diesen Daten können z.B. auch typische Parameter wie Vorneigung, Seitenneigung, usw., der Brille bestimmt werden.

Bei dem Verfahren zur Zentrierdatenermittlung kann die bestimmte tatsächliche momentane Kopfhaltung mit Hilfe der bestimmten habituellen Kopfhaltung derart korrigiert werden, dass die Zentrierdaten auf Basis der habituellen und nicht der zufälligen Kopfhaltung bestimmt werden. Die Korrektur kann zum Beispiel erfolgen, indem die Abweichung der momentanen Kopfhaltung von der habituellen Kopfhaltung ermittelt wird und anhand dieser Abweichung eine Umrechnung/Korrektur der Zentrierwerte erfolgt.

Die ermittelte habituelle Kopfhaltung des Probanden/Kunden und der ggf. ebenfalls ermittelte Vorneigungswinkel der Brille können auch zur erzwungenen Ausrichtung des Kunden für die konventionelle Zentrierdatenermittlung dienen, indem bei Erreichen des angestrebten Vorneigungswinkels der Brille eine automatische Auslösung der bildgebenden Einheiten eingeleitet wird bzw. ein Signal dem Operator gegeben wird, dass nun die Zentrierdatenermittlung erfolgen kann.

Die Erfindung sieht insbesondere auch vor, dass die vorstehend beschriebenen Verfahren zur Ermittlung der habituellen Kopfhaltung sowie zur Zentrierdatenermittlung in Form von in einem Computer ausführbaren Computerprogrammen mit Programmcodes vorliegen. Die Computerprogramme können auf einem maschinenlesbaren Datenträger gespeichert sein.

Der Vollständigkeit halber wird darauf hingewiesen, dass die o.a. Aufzeichnungseinrichtung zur Aufzeichnung der Kopfhaltung über eine Zeitspanne ein am Kopf des Probanden anbringbares Positionsaufzeichnungsgerät und/oder ein Bildaufzeichnungsgerät umfassen kann.
Positionsaufzeichnungsgerät und/oder Bildaufzeichnungsgerät können z.B. in der vorstehend beschriebenen Weise ausgebildet sein. Die Ermittlungseinrichtung zur Ermittlung einer bevorzugten Kopfhaltung aus der Aufzeichnung kann einen Computer umfassen.

Die Kopfhaltungsbestimmungseinrichtung zur Bestimmung der tatsächlichen momentanen Kopfhaltung eines Probanden kann ein Positionsaufzeichnungsgerät und/oder ein Bildaufzeichnungsgerät, insbesondere der vorstehend beschriebenen Art, umfassen.

Die Ermittlungseinrichtung zur Ermittlung der Anordnung einer Brillenfassung relativ zur bestimmten tatsächlichen momentanen Kopfhaltung kann ein Bildaufzeichnungsgerät, insbesondere eine Video- oder Digitalkamera, und/oder einen Computer umfassen.

Die Erfindung wird nunmehr anhand der Zeichnung näher beschrieben. Es zeigen:
- Figur 1: eine schematische Darstellung einer erfindungsgemäßen Vorrichtung zur Zentrierdatenermittlung mit erfindungsgemäßer Vorrichtung zur Ermittlung der habituellen Kopfhaltung eines Probanden;
- Figur 2: Aufnahmen des Gesichts eines Kunden mit angepasster Fassung in Nullblickrichtung
a. von vorn
b. von der Seite
c. von vorn mit Zentrierdaten, nämlich x, y
d. von der Seite mit Zentrierdaten, nämlich Hornhautscheitelabstand und Fassungsvorneigung

Es wird angenommen, dass ein Kunde ein Optikergeschäft zum Zwecke des Erwerbs einer neuen Brille aufsucht. Zunächst wird er hinsichtlich der für ihn in Frage kommenden Brillengläser und Fassungen beraten. Nach erfolgter Auswahl der Fassung erfolgt die eigentliche Zentrierdatenermittlung mit Hilfe einer erfindungsgemäßen Vorrichtung.

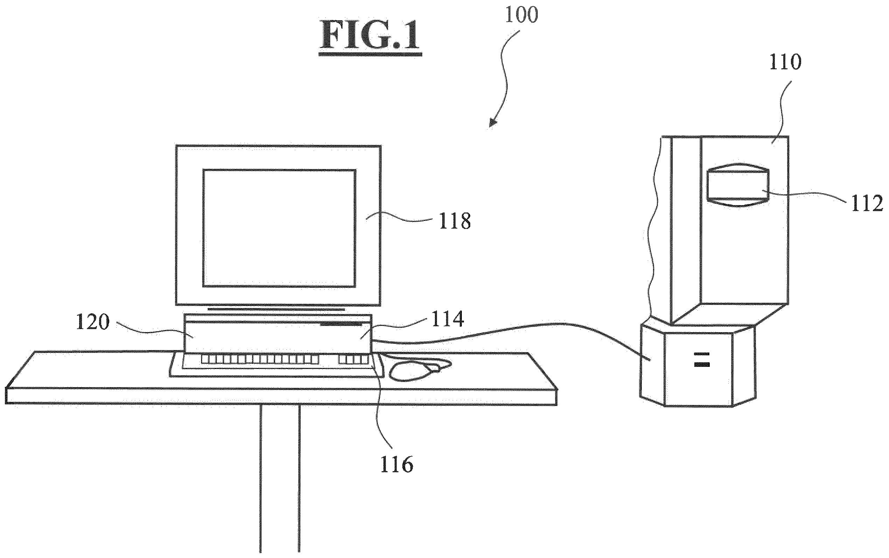

Die Figur 1 zeigt eine erfindungsgemäße Vorrichtung zur Zentrierdatenermittlung 100. Diese Vorrichtung zur Zentrierdatenermittlung 100 umfasst ein Videozentriergerät 110 mit Videokamera 112 und Computer 114 mit Tastatur 116, Bildschirm 118 und Speichermedium 120. Es handelt sich um ein kalibriertes System, also ein System zur Aufnahme und Bestimmung von Abmessungen.

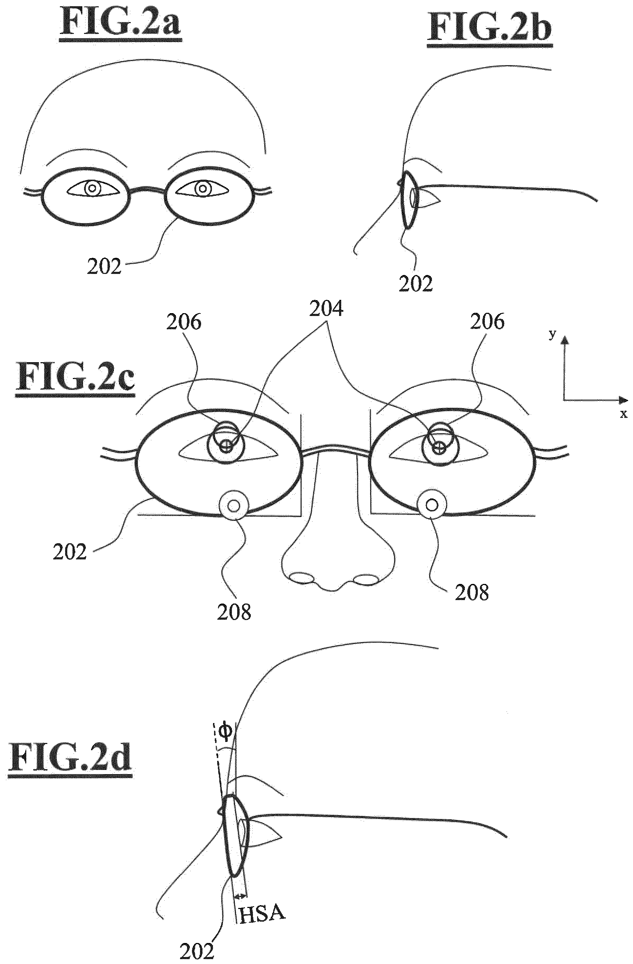

Das Gesicht des Kunden wird mit angepasster Fassung 202 in Nullblickrichtung von vorn und von der Seite mit der Videokamera 112 aufgenommen, wie dies in den Figuren 2a) und b) dargestellt ist.

Mit der auf den Bildschirm 118 dargestellten Frontaufnahme (Figur 2c) wird zunächst die Fassungsform 202 erfasst. Weiterhin werden die Pupillenmitten 204, z.B. im Koinzidenzverfahren, relativ zur Fassungsform 202 ermittelt. Mit der Seitenaufnahme werden der Hornhautscheitelabstand HSA und die Fassungsvorneigung ϕ zur Erfüllung der Augendrehpunktforderung ermittelt, wie dies in Figur 2d) dargestellt ist.

Erfindungsgemäß ist nunmehr vorgesehen, die habituelle Kopfhaltung und die Neigung der Brille z.B. mit Hilfe der kalibrierten Videokamera über einen längeren Zeitraum aufzuzeichnen und hieraus eine habituelle Kopfhaltung und eine bevorzugte Neigung der Brille zu ermitteln.

Die somit ermittelte Kopfhaltung und der damit zusammenhängende Vorneigungswinkel der Brille sowie andere Messdaten wie z.B. die Anordnung der Brille in der Fassungs- oder Scheibenebene in x, y dienen zur Umrechnung der mittels des Videozentriergeräts 110 konventionell ermittelten Zentrierdaten (z.B. Fernbezugspunkt 206, Nahmesskreis 208), falls bei deren Bestimmung der Kunde eine von der normalen Kopfhaltung abweichende Kopfhaltung eingenommen hatte (vgl. Figur 2c).

Der Gegenstand der Erfindung wird nachfolgend in Form von Klauseln im Sinne der J 15/88 wiedergegeben:
1. Verfahren zur Ermittlung der habituellen Kopfhaltung eines Probanden, gekennzeichnet durch die Schritte:
   a. Aufzeichnung der Kopfhaltung über eine Zeitspanne;
   b. Ermittlung einer bevorzugten Kopfhaltung aus der Aufzeichnung.
2. Verfahren nach Klausel 1, dadurch gekennzeichnet, dass die Zeitspanne vorgegeben wird.
3. Verfahren nach einer der vorangegangenen Klauseln, dadurch gekennzeichnet, dass als bevorzugte Kopfhaltung eine Kopfhaltung gewählt wird, in der der Proband seinen Kopf bei einer vorgegebenen Sehaufgabe mit einer Wahrscheinlichkeit von über 20 %, insbesondere von über 50 %, hält.
4. Verfahren nach einer der vorangegangenen Klauseln, dadurch gekennzeichnet, dass als bevorzugte Kopfhaltung die aufgezeichnete Haltung gewählt wird, in der der Proband den Kopf bei einer vorgegebenen Sehaufgabe mit der größten Wahrscheinlichkeit hält.
5. Verfahren nach einer der vorangegangenen Klauseln, dadurch gekennzeichnet, dass als bevorzugte Kopfhaltung eine der aufgezeichneten Haltungen gewählt wird, in der der Proband den Kopf beim Blick in die Nähe und/oder beim Blick in die Ferne hält.
6. Verfahren nach einer der vorangegangenen Klauseln, dadurch gekennzeichnet, dass als bevorzugte Kopfhaltung eine der aufgezeichneten Haltungen gewählt wird, während der der Proband steht und/oder sitzt.
7. Verfahren nach einer der vorangegangenen Klauseln, dadurch gekennzeichnet, dass dem Probanden Alltagssituationen und/oder alltägliche Sehaufgaben dargeboten werden.
8. Verfahren nach einer der vorangegangenen Klauseln, dadurch gekennzeichnet, dass die Aufzeichnung der Kopfhaltung mittels eines am Kopf des Probanden angebrachten Positionsaufzeichnungsgeräts durchgeführt wird.
9. Verfahren nach einer der vorangegangenen Klauseln, dadurch gekennzeichnet, dass die Aufzeichnung der Kopfhaltung mittels eines Bildaufzeichnungsgeräts (110) durchgeführt wird.
10. Verfahren zur Zentrierdatenermittlung, bei dem
   a. eine tatsächliche momentane Kopfhaltung eines Probanden bestimmt wird,
   b. eine Anordnung einer Brillenfassung (202) relativ zur bestimmten tatsächlichen momentanen Kopfhaltung ermittelt wird,
      dadurch gekennzeichnet, dass
   c. mit Hilfe eines automatisierten Verfahrens eine habituelle Kopfhaltung bestimmt wird und
   d. die bestimmte tatsächliche momentane Kopfhaltung mit Hilfe der bestimmten habituellen Kopfhaltung korrigiert wird.
11. Verfahren nach Klausel 10, dadurch gekennzeichnet, dass in Schritt c. die Neigung (ϕ) der Brillenfassung (202) mit bestimmt wird.
12. Verfahren nach einer der Klauseln 10 oder 11, dadurch gekennzeichnet, dass die habituelle Kopfhaltung mit einem Verfahren nach einem der Ansprüche 1 bis 9 bestimmt wird.
13. Verfahren nach einer der Klauseln 10 bis 12, dadurch gekennzeichnet, dass die bestimmte tatsächliche momentane Kopfhaltung mit Hilfe der bestimmten habituellen Kopfhaltung derart korrigiert wird, dass die Zentrierdaten (ϕ, HSA, x, y) auf Basis der habituellen und nicht der zufälligen Kopfhaltung bestimmt werden.
14. Verfahren nach Klausel 13, dadurch gekennzeichnet, dass die bestimmte tatsächliche momentane Kopfhaltung mit Hilfe der bestimmten habituellen Kopfhaltung korrigiert wird, indem die Abweichung der momentanen Kopfhaltung zur habituellen Kopfhaltung ermittelt wird und anhand dieser eine Umrechnung der Zentrierwerte erfolgt.
15. Computerprogramm mit Programmcode zur Durchführung des Verfahrens nach einer der vorangegangenen Klauseln, wenn das Programm in einem Computer (114) ausgeführt wird.
16. Computerprogramm nach Klausel 15, gespeichert auf einem maschinenlesbaren Datenträger (120).
17. Vorrichtung (100) zur Ermittlung der habituellen Kopfhaltung eines Probanden mit:
   a. einer Aufzeichnungseinrichtung (110) zur Aufzeichnung der Kopfhaltung über eine Zeitspanne;
   b. Ermittlungseinrichtung (114) zur Ermittlung einer bevorzugten Kopfhaltung aus der Aufzeichnung.
18. Vorrichtung nach Klausel 17, dadurch gekennzeichnet, dass die Aufzeichnungseinrichtung ein am Kopf des Probanden anbringbares Positionsaufzeichnungsgerät und/oder ein Bildaufzeichnungsgerät (112) umfasst.
19. Vorrichtung nach einer der Klauseln 17 oder 18, dadurch gekennzeichnet, dass die Ermittlungseinrichtung einen Computer (114) umfasst.
20. Vorrichtung (100) zur Zentrierdatenermittlung mit
   a. einer Kopfhaltungsbestimmungseinrichtung (110), um eine tatsächliche momentane Kopfhaltung eines Probanden zu bestimmen,
   b. einer Ermittlungseinrichtung (114), um eine Anordnung einer Brillenfassung relativ zur bestimmten tatsächlichen momentanen Kopfhaltung zu ermitteln, dadurch gekennzeichnet, dass
   c. eine Vorrichtung (110) zur automatischen Bestimmung der habituellen Kopfhaltung und
   d. eine Korrektureinrichtung (114) zur Korrektur der bestimmten tatsächlichen momentanen Kopfhaltung mit Hilfe der bestimmten habituellen Kopfhaltung vorgesehen sind.
21. Vorrichtung nach Klausel 20, dadurch gekennzeichnet, dass die Kopfhaltungsbestimmungseinrichtung ein Positionsaufzeichnungsgerät und/oder ein Bildaufzeichnungsgerät (112) umfasst.
22. Vorrichtung nach einer der Klauseln 20 oder 21, dadurch gekennzeichnet, dass die Ermittlungseinrichtung ein Bildaufzeichnungsgerät (112) und/oder einen Computer (114) umfasst.
23. Vorrichtung nach einer der Klauseln 20 bis 22, dadurch gekennzeichnet, dass die Vorrichtung zur Bestimmung der habituellen Kopfhaltung nach einer der Klauseln 17 bis 19 ausgebildet ist.

## Patentansprüche

1. Verfahren zur Zentrierdatenermittlung, bei dem
a. eine tatsächliche momentane Kopfhaltung eines Probanden bestimmt wird,
b. eine Anordnung einer Brillenfassung (202) relativ zur bestimmten tatsächlichen momentanen Kopfhaltung ermittelt wird,
c. mit Hilfe eines automatisierten Verfahrens eine habituelle Kopfhaltung bestimmt wird, und
d. die bestimmte tatsächliche momentane Kopfhaltung mit Hilfe der bestimmten habituellen Kopfhaltung korrigiert wird, wobei die bestimmte tatsächliche momentane Kopfhaltung mit Hilfe der bestimmten habituellen Kopfhaltung derart korrigiert wird, dass die Zentrierdaten auf Basis der habituellen und nicht der zufälligen Kopfhaltung bestimmt werden, **dadurch gekennzeichnet, dass**
e. die Bestimmung der habituellen Kopfhaltung durch Ermittlung einer bevorzugten Kopfhaltung aus Daten einer Aufzeichnung der Kopfhaltung über eine Zeitspanne, während der der Proband abgelenkt ist, erfolgt, und dass
f. die bestimmte tatsächliche momentane Kopfhaltung mit Hilfe der bestimmten habituellen Kopfhaltung korrigiert wird, indem die Abweichung der momentanen Kopfhaltung zur habituellen Kopfhaltung ermittelt wird und anhand dieser eine Umrechnung der Zentrierwerte erfolgt.

2. Verfahren nach Anspruch 1, **dadurch gekennzeichnet, dass** dem Probanden zur dynamischen Ermittlung der habituellen Kopfhaltung über eine Zeitspanne Alltagssituationen bzw. alltägliche Sehaufgaben im Nah- und/oder Fernbereich dargeboten werden und in dieser Zeitspanne die Kopfhaltung fortlaufend gemessen wird; und dass als bevorzugte Kopfhaltung die aufgezeichnete Haltung gewählt wird, in der der Proband den Kopf bei einer vorgegebenen Sehaufgabe mit der größten Wahrscheinlichkeit hält.

3. Verfahren nach Anspruch 2, **dadurch gekennzeichnet, dass** die Zeitspanne vorgegeben wird.

4. Verfahren nach einem der vorangegangenen Ansprüche, **dadurch gekennzeichnet, dass** als bevorzugte Kopfhaltung eine Kopfhaltung gewählt wird, in der der Proband seinen Kopf bei einer vorgegebenen Sehaufgabe mit einer Wahrscheinlichkeit von über 20 % oder von über 50 % hält.

5. Verfahren nach einem der vorangegangenen Ansprüche, **dadurch gekennzeichnet, dass** als bevorzugte Kopfhaltung eine der aufgezeichneten Haltungen gewählt wird, in der der Proband den Kopf beim Blick in die Nähe und/oder beim Blick in die Ferne hält.

6. Verfahren nach einem der vorangegangenen Ansprüche, **dadurch gekennzeichnet, dass** als bevorzugte Kopfhaltung eine der aufgezeichneten Haltungen gewählt wird, während der der Proband steht oder sitzt.

7. Verfahren nach einem der vorangegangenen Ansprüche, **dadurch gekennzeichnet, dass** in Schritt c. die Neigung (φ) der Brillenfassung (202) mit bestimmt wird

8. Verfahren nach einem der vorangegangenen Ansprüche, **dadurch gekennzeichnet, dass** die Aufzeichnung der Kopfhaltung mittels eines am Kopf des Probanden angebrachten Positionsaufzeichnungsgeräts durchgeführt wird.

9. Verfahren nach einem der vorangegangenen Ansprüche, **dadurch gekennzeichnet, dass** die Aufzeichnung der Kopfhaltung mittels eines Bildaufzeichnungsgeräts (110) durchgeführt wird.

10. Vorrichtung (100) zur Zentrierdatenermittlung mit
a. einer Kopfhaltungsbestimmungseinrichtung (110), um eine tatsächliche momentane Kopfhaltung eines Probanden zu bestimmen,
b. einer Ermittlungseinrichtung (114), um eine Anordnung einer Brillenfassung relativ zur bestimmten tatsächlichen momentanen Kopfhaltung zu ermitteln,
c. eine Vorrichtung (110) zur automatischen Bestimmung der habituellen Kopfhaltung, und
d. eine Korrektureinrichtung (114) zur Korrektur der bestimmten tatsächlichen momentanen Kopfhaltung mit Hilfe der bestimmten habituellen Kopfhaltung, wobei die bestimmte tatsächliche momentane Kopfhaltung mit Hilfe der bestimmten habituellen Kopfhaltung derart korrigiert wird, dass die Zentrierdaten auf Basis der habituellen und nicht der zufälligen Kopfhaltung bestimmt werden,
**dadurch gekennzeichnet, dass**
e. die Vorrichtung (110) zur automatischen Bestimmung der habituellen Kopfhaltung durch Ermittlung einer bevorzugten Kopfhaltung aus Daten einer Aufzeichnung der Kopfhaltung über eine Zeitspanne, während der der Proband abgelenkt ist, ausgebildet ist, und dass
f. die Korrektureinrichtung (114) zur Korrektur der bestimmten tatsächlichen momentanen Kopfhaltung mit Hilfe der bestimmten habituellen Kopfhaltung ausgebildet ist, indem die Abweichung der momentanen Kopfhaltung zur habituellen Kopfhaltung ermittelt wird und anhand dieser eine Umrechnung der Zentrierwerte erfolgt.

11. Vorrichtung nach Anspruch 10, **dadurch gekennzeichnet, dass** die Kopfhaltungsbestimmungseinrichtung (110) ein am Kopf des Probanden anbringbares Positionsaufzeichnungsgerät und/oder ein Bildaufzeichnungsgerät (112) umfasst.

12. Vorrichtung nach einem der Ansprüche 10 oder 11, **dadurch gekennzeichnet, dass** die Vorrichtung (110) zur automatischen Bestimmung der habituellen Kopfhaltung einen Computer (114) umfasst.

13. Computerprogramm mit Programmcode zur Durchführung des Verfahrens nach einem der vorangegangen Ansprüche 1 bis 9, wenn das Programm in einem Computer (114) der Ermittlungseinrichtung einer Vorrichtung nach einem der Ansprüche 10 bis 12 ausgeführt wird.
